# EUROPEAN PATENT APPLICATION

(11) **EP 1 557 416 A1**
(43) Date of publication of application: **27.07.2005**
(21) Application number: 04001506.7
(22) Date of filing: 23.01.2004
(51) Int. Cl.: C07D 471/04, C07D 413/14, C07D 413/12, A61K 31/422, A61K 31/435, A61K 31/4375, A61P 31/04

(54) **Oxazolidinone-quinolone hybrid antibiotics**

(71) Applicant: Morphochem Aktiengesellschaft Für Kombinatorische Chemie, 81379 München (DE)
(72) Inventor: Hubschwerlen, Christian, Durmenach (FR); Specklin, Jean-Luc, 68680 Kembs-Schaeferhof (FR); Baeschlin, Daniel Kaspar, 4144 Arlesheim (CH); Sigwalt, Christine, 68320 Kunheim (FR); Mueller, Stefan, Altdorf (DE); Cappi, Michael, 81369 München (DE)
(74) Representative: Forstmeyer, Dietmar, Dr. rer. nat., Dipl.-Chem.

(57) **Abstract**

The present invention relates to compounds of the Formula (I) that are useful antimicrobial agents and effective ag ainst a variety of multi-drug resistant bacteria:

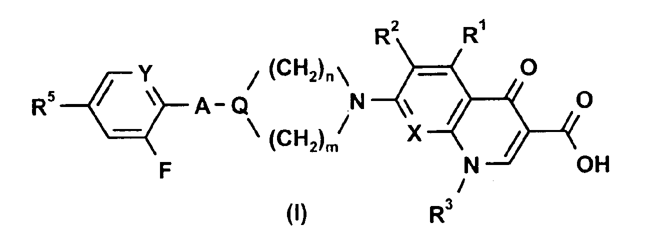

## Description

The present invention describes new compounds in which the pharmacophores of quinolone and oxazolidinone are linked together through a linker that is stable under physiological conditions and a pharmaceutical antibacterial composition containing these compounds. These dual action compounds are useful antimicrobial agents effective against a variety of human and veterinary pathogens including Gram positive aerobic bacteria such as multiply-resistant staphylococci, streptococci and enterococci as well as Gram negative bacteria such as Moraxella catarrhalis and Haemophilus influenzae and anaerobic organisms such as bacteroides spp. and Clostridia spp. species and acid-fast organism such as Mycobacterium tuberculosis, Mycobacterium avium spp.

Oxazolidinone-quinolone hybrid antibiotics have already been described (WO02059116, W003002560, W003031443, W003032962). The major drawback of the compounds known in the state of the art is the poor water solubility, which makes the development of a formulation difficult.

The present invention provides new compounds of formula (I), that are useful antimicrobial agents and effective against a variety of multi-drug resistant bacteria 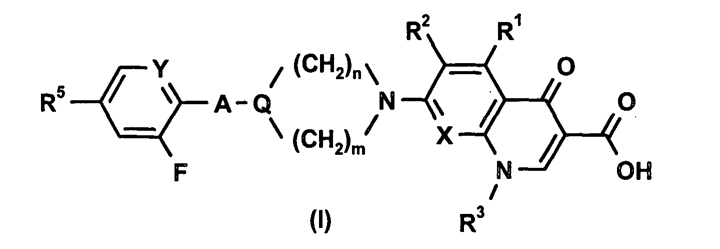 wherein
A is an alkylene group, an alkenylene group, an alkynylene group, a heteroalkylene group, a cycloalkylene group, a heterocycloalkylene group, an arylene group or a heteroarylene group all of which groups may be substituted;
Q is CR⁴ or N;
X is CR⁷ or N;
Y is CR⁶ or N;
n is 1, 2 or 3;
m is 1, 2 or 3;
R¹ is H, F, Cl, Br, I, OH, NH₂, an alkyl group or a heteroalkyl group;
R² is H, F or Cl;
R³ is H, an alkyl group, an alkenyl group, an alkynyl group, a heteroalkyl group, a cycloalkyl group, a heterocycloalkyl group, an aryl group, a heteroaryl group, an alkylaryl group or a heteroarylalkyl group; all of which may be substituted with one, two or more halogen atoms like F or Cl or amino groups.
R⁴ is hydrogen, hydroxy, a group of formula OPO₃H₂ or OSO₃H or a heteroalkyl group carrying at least one OH, NH₂, SO₃H, PO₃H₂ or COOH group;
R⁵ is selected from following groups: 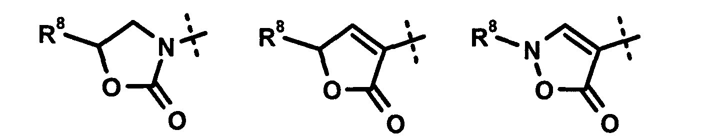 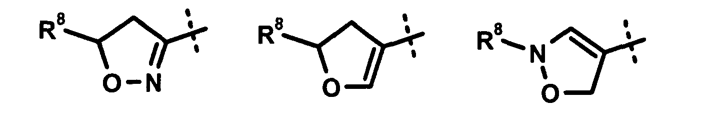 R⁶ is H, F, Cl or OMe;
R⁷ is H, F, Cl, OH, NH₂, an alkyl group or a heteroalkyl group, or
R³ and R⁷ can be linked via an alkylene, an alkenylene or a heteroalkylene group or be a part of a cycloalkylene or heterocycloalkylene group; in case R³ is no H and R⁵ is no H, F, OH, NH₂ or Cl; and
R⁸ is a C₁₋₆ heteroalkyl or a heteroarylalkyl group;
or a pharmacologically acceptable salt, solvate, hydrate or formulation thereof.

It should be appreciated that certain compounds of formula (I) may have tautomeric forms from which only one might be specifically mentioned or depicted in the following description, different geometrical isomers (which are usually denoted as cis/trans isomers or more generally as (E) and (Z) isomers) or different optical isomers as a result of one or more chiral carbon atoms (which are usually nomenclatured under the Cahn-Ingold-Prelog or R/S system). Further, some compounds may display polymorphism. All these tautomeric forms, geometrical or optical isomers (as well as racemates and diastereomers) and polymorphous forms are included in the invention.

The term alkyl refers to a saturated or unsaturated (i.e. alkenyl and alkinyl) straight or branched chain alkyl group, containing from one to ten, preferably one to six carbon atoms for example methyl, ethyl, propyl, isopropyl, butyl, isobutyl, sec-butyl, tert-butyl, n-pentyl, iso-pentyl, n-hexyl, 2,2-dimethylbutyl, n-octyl; ethenyl (vinyl), propenyl (allyl), iso-propenyl, n-pentyl, n-pentenyl, butenyl, isoprenyl or hexa-2-enyl; ethinyl, propinyl or butinyl groups. Any alkyl group as defined herein may be substituted with one, two or more substituents, for example F, Cl, Br, I, NH₂, OH, SH or NO₂.

The terms alkenyl and alkynyl refer to a unsaturated straight or branched chain alkyl group (having one, two or more double and/or triple bonds, an alkenyl preferably having one or two double bonds and an alkynyl preferably having one or two triple bonds), containing from two to ten, preferably two to six carbon atoms for example: ethenyl (vinyl), propenyl (allyl), iso-propenyl, n-pentenyl, butenyl, isoprenyl or hexa-2-enyl; ethynyl, propynyl or butynyl groups. Any alkenyl or alkynyl group as defined herein may be substituted with one, two or more substituents, for example F, Cl, Br, I, NH₂, OH, SH or NO₂.

The term heteroalkyl refers to an alkyl, alkenyl or alkynyl group as defined herein where one or more carbon atoms are replaced by an oxygen, nitrogen, phosphorous or sulphur atom for example an alkoxy group such as methoxy, ethoxy, propoxy, iso-propoxy, butoxy or tert.-butoxy, an alkoxyalkyl group such as methoxymethyl, ethoxymethyl, 1-methoxyethyl, 1-ethoxyethyl, 2-methoxyethyl or 2-ethoxyethyl, an alkylamino group such as methylamino, ethylamino, propylamino, isopropylamino, dimethylamino or diethylamino, an alkylthio group such as methylthio, ethylthio or isopropylthio or a cyano group. It may also refer to one of the above groups containing a keto group. The term heteroalkyl furthermore refers to a group derived from a carboxylic acid or carboxylic acid amide such as acetyl, propionyl, acetyloxy, propionyloxy, acetylamino or propionylamino, a carboxyalkyl group such as carboxymethyl, carboxyethyl or carboxypropyl, a carboxyalkyl ester, an alkylthiocarboxyamino group, an alkoxyimino group, an alkylaminothiocarboxyamino group or an alkoxycarbonylamino group. Any heteroalkyl group as defined herein may be substituted with one, two or more substituents, for example F, Cl, Br, I, NH₂, OH, SH or NO₂.

The term cycloalkyl refers to a saturated or partially unsaturated (having one, two or more double and/or triple bonds), cyclic group with one, two or more rings, having three to 14 carbon ring-atoms, preferably from five or six to ten carbon ring-atoms, for example cyclopropyl, cyclobutyl, cyclopentyl, cyclohexyl, tetralin, cyclopentenyl or cyclohex-2-enyl groups. Any cycloalkyl group as defined herein may be substituted with one, two or more substituents, for example F, Cl, Br, I, OH, NH₂, SH, N₃, NO₂, alkyl groups such as methyl or ethyl, heteroalkyl groups such as methoxy, methylamino, dimethylamino or cyanide.

The term heterocycloalkyl refers to a cycloalkyl group as defined herein where one, two or more carbon ring-atoms are replaced by one, two or more oxygen, nitrogen, phosphorous or sulphur atoms or S(0)₁₋₂ groups for example piperidino, morpholino or piperazino groups.

The term aryl refers to an aromatic cyclic group with one, two or more rings, having five to 14 carbon ring-atoms preferably from five or six to ten carbon ring-atoms, for example phenyl or naphthyl groups. Any aryl group as defined herein may be substituted with one, two or more substituents, for example F, Cl, Br, I, OH, NH₂, SH, N₃, NO₂, alkyl groups such as methyl or ethyl, heteroalkyl groups such as methoxy, methylamino, dimethylamino or cyanide.

The term heteroaryl refers to an aryl group as defined herein where one, two or more ring-carbon atoms are replaced by an oxygen, nitrogen, boron, phosphorous or sulphur atom, for example pyridyl, imidazolyl, pyrazolyl, quinolinyl, isoquinolinyl, pyrrolyl, oxazolyl, isoxazolyl, thiazolyl, isothiazolyl, 1,2,3-triazolyl, 1,2,4-triazolyl, oxadiazolyl, thiadiazolyl, indolyl, indazolyl, tetrazolyl, pyrazinyl, pyrimidinyl and pyridazinyl groups.

The terms arylalkyl, alkylaryl and heteroarylalkyl, heteroalkylaryl refer to groups that comprise both aryl or, respectively, heteroaryl as well as alkyl and/or heteroalkyl and/or cycloalkyl and/or heterocycloalkyl groups.
Any alkyl, alkenyl, alkynyl, heteroalkyl, cycloalkyl, heterocycloalkyl, aryl, heteroaryl aralkyl or heteroarylalkyl groups as defined herein may be substituted with one or more halogen atoms, NH₂, SH, NO₂ or OH groups or unsubstituted alkyl, heteroalkyl, aryl, aralkyl, aralkyloxy, heteroaryl, cycloalkyl or heterocycloalkyl groups as defined herein.

The term "optionally substituted" or "substituted" refer to groups wherein one or more hydrogen atoms may be replaced a halogen atom, a NH₂, SH, NO₂ or OH group or by an unsubstituted alkyl, heteroalkyl, aryl, aralkyl, aralkyloxy, heteroaryl, cycloalkyl or heterocycloalkyl group as defined herein.

Preferred and/or advantageous embodiments of the invention are subject-matter of the subclaims.

Preferred are compounds of Formula (I), wherein R¹ is H.

Further preferred are compounds of Formula (I), wherein R² is F or H.

Moreover preferred are compounds of Formula (I), wherein R³ is an ethyl, a 2-propyl, a C₃-C₆ cycloalkyl, a phenyl or a pyridyl group. All these groups may be substituted with one, two or more fluorine atoms or amino groups.

Moreover preferred are compounds of Formula (I), wherein R³ is a cyclopropyl group.

Further preferred are compounds of Formula (I), wherein R³ and R⁷ together form a bridge of the formula -O-CH₂-N(Me)- or -O-CH₂-CH(Me)-. Herein, the preferred stereochemistry at the chiral center is the one giving the S configuration in the final compound.

Moreover preferred are compounds of formula (I), wherein R⁴ is hydroxy or a group of formula OSO₃H, OPO₃H₂, OCH₂OPO₃H₂, OCOCH₂CH₂COOH or an ester of a naturally occurring amino acid or a derivative thereof (i.e dimethyl aminoglycine).

Further preferred are compounds of Formula (I), wherein R⁸ is a group of the formula -CH₂NHCOCH=CHAryl, - CH₂Oheteroaryl (especially -oxa-3-oxazol), -CH₂NHSO₂Me, -CH₂NHCOOMe, -CH₂NHCS₂Me, -CH₂NHCSNH₂, -CH₂NHCSOMe or -NHCOMe.

Especially preferred are compounds of Formula (I), wherein R⁵ has the following structure:

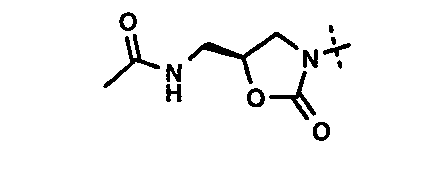

Moreover preferred are compounds of Formula (I), wherein R⁷ is H, F, Cl or a methoxy group which may be substituted by one, two or three fluorine atoms.

Further preferred are compounds of formula (I), wherein X is N or CH.

Moreover preferred are compounds of Formula (I), wherein Y is CH.

Further preferred are compounds of Formula (I), wherein A is C₁₋₆ alkyl, C₂₋₆ alkenyl, C₂₋₆ alkynyl, C₁₋₆ heteroalkyl, cyclopropyl, epoxide, aziridine, thioepoxide, lactame or lactone, all of which groups may be subsitituted.

Especially preferred are compounds of formula (I), wherein A is a group of formula -CH=CH-, -C≡C-, -CH(OH)CH(OH)-or -CH(NH₂)CH(OH) -.

The present invention also relates to pharmacologically acceptable salts, or solvates and hydrates, respectively, and to compositions and formulations of compounds of Formula (I). The present invention describes procedures to produce pharmaceutically useful agents, which contain these compounds, as well as the use of these compounds for the production of pharmaceutically useful agents.

The pharmaceutical compositions according to the present invention contain at least one compound of Formula (I) as the active agent and optionally carriers and/or diluents and/or adjuvants. Optionally the pharmaceutical compositions according to the present invention may also contain additional known antibiotics.

Examples of pharmacologically acceptable salts of sufficiently basic compounds of Formula (I) are salts of physiologically acceptable mineral acids like hydrochloric, hydrobromic, sulfuric and phosphoric acid; or salts of organic acids like methanesulfonic, p-toluenesulfonic, lactic, acetic, trifluoroacetic, citric, succinic, fumaric, maleic and salicylic acid. Further, a sufficiently acidic compound of Formula (I) may form alkali or earth alkaline metal salts, for example sodium, potassium, lithium, calcium or magnesium salts; ammonium salts; or organic base salts, for example methylamine, dimethylamine, trimethylamine, triethylamine, ethylenediamine, ethanolamine, choline hydroxide, meglumin, piperidine, morpholine, tris-(2-hydroxyethyl)amine, lysine or arginine salts. Compounds of Formula (I) may be solvated, especially hydrated. The hydratisation can occur during the process of production or as a consequence of the hygroscopic nature of the initially water free compounds of Formula (I). The compounds of Formula (I) contain asymmetric C-atoms and may be present either as achiral compounds, mixtures of diastereomers, mixtures of enantiomers or as optically pure compounds.

The present invention also relates to pro-drugs which are composed of a compound of Formula (I) and at least one pharmacologically acceptable protective group which will be cleaved off under physiological conditions, such as an alkoxy-, aralkyloxy-, acyl-, SO₃H, PO₃H₂, acyloxymethyl group (e.g. pivaloyloxymethyl), an 2-alkyl-, 2-aryl- or 2-aralkyloxycarbonyl-2-alkylidene ethyl group or an acyloxy group as defined herein, e.g. ethoxy, benzyloxy, acetyl or acetyloxy. Especially preferred are prodrugs of the hydroxy group of a compound of formula (I) wherein R⁴ is H.

As mentioned above, therapeutically useful agents that contain compounds of Formula (I), their solvates, salts or formulations are also comprised in the scope of the present invention. In general, compounds of Formula (I) will be administered by using the known and acceptable modes known in the art, either alone or in combination with any other therapeutic agent. Such therapeutically useful agents can be administered by one of the following routes: oral, e.g. as tablets, dragees, coated tablets, pills, semisolids, soft or hard capsules, for example soft and hard gelatine capsules, aqueous or oily solutions, emulsions, suspensions or syrups, parenteral including intravenous, intramuscular and subcutaneous injection, e.g. as an injectable solution or suspension, rectal as suppositories, by inhalation or insufflation, e.g. as a powder formulation, as microcrystals or as a spray (e.g. liquid aerosol), transdermal, for example via an transdermal delivery system (TDS) such as a plaster containg the active ingredient or intranasal. For the production of such tablets, pills, semisolids, coated tablets, dragees and hard, e.g. gelatine, capsules the therapeutically useful product may be mixed with pharmaceutically inert, inorganic or organic excipients as are e.g. lactose, sucrose, glucose, gelatin, malt, silica gel, starch or derivatives thereof, talc, stearinic acid or their salts, dried skim milk, and the like. For the production of soft capsules one may use excipients as are e.g. vegetable, petroleum, animal or synthetic oils, wax, fat, polyols. For the production of liquid solutions, emulsions or suspensions or syrups one may use as excipients e.g. water, alcohols, aqueous saline, aqueous dextrose, polyols, glycerin, lipids, phospholipids, cyclodextrins, vegetable, petroleum, animal or synthetic oils. Especially preferred are lipids and more preferred are phospholipids (preferred of natural origin; especially preferred with a particle size between 300 to 350 nm) preferred in phosphate buffered saline (pH = 7 to 8, preferred 7.4). For suppositories one may use excipients as are e.g. vegetable, petroleum, animal or synthetic oils, wax, fat and polyols. For aerosol formulations one may use compressed gases suitable for this purpose, as are e.g. oxygen, nitrogen and carbon dioxide. The pharmaceutically useful agents may also contain additives for conservation, stabilisation, e.g. UV stabilizers, emulsifiers, sweetener, aromatisers, salts to change the osmotic pressure, buffers, coating additives and antioxidants.

A daily dosage per patient of about 1mg to about 4000mg especially about 50mg to 3 g is usual with those of ordinary skill in the art appreciating that the dosage will depend also upon the age, conditions of the mammals, and the kind of diseases being treated or prevented. The daily dosage can be administrated in a single dose or can be divided over several doses. An average single dose of about 50mg, 100mg, 250mg, 500mg, 1000mg and 2000mg can be contemplated.

### Examples

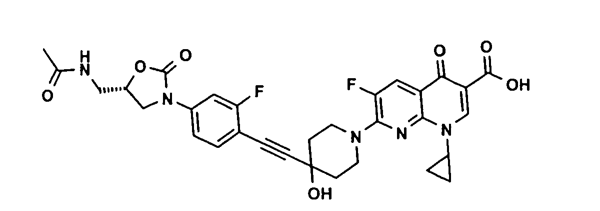

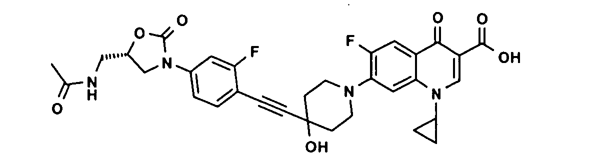

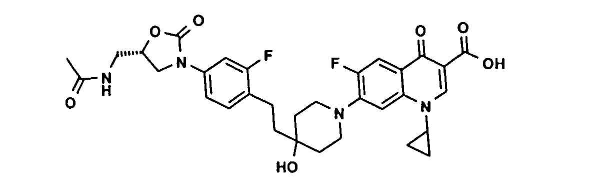

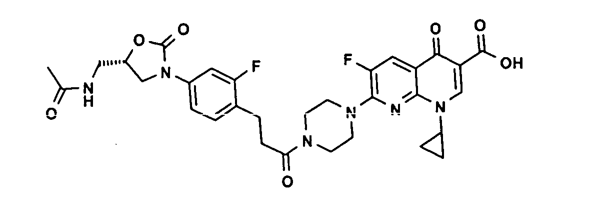

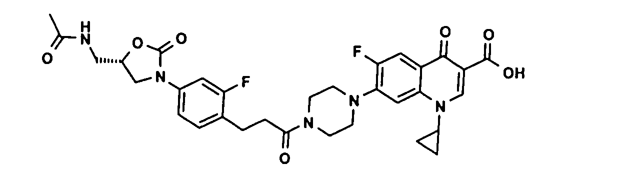

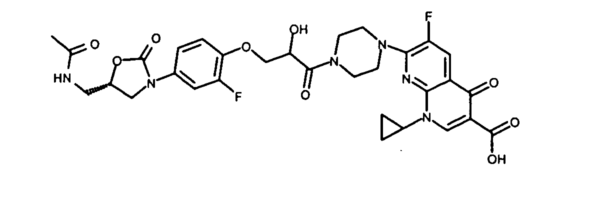

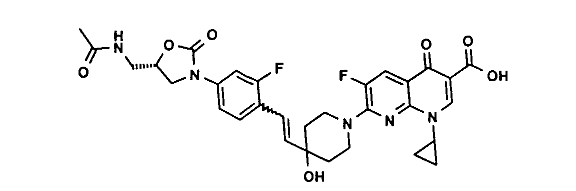

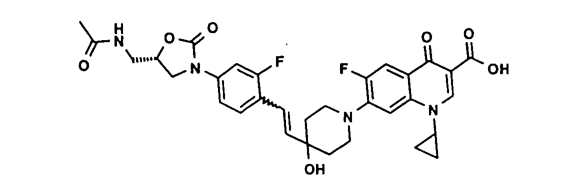

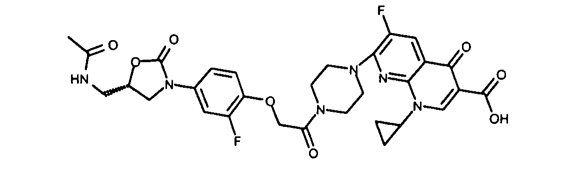

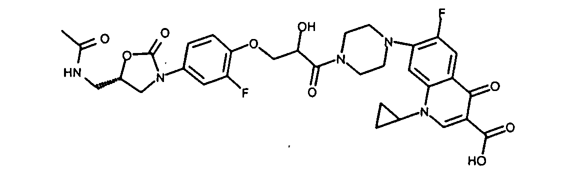

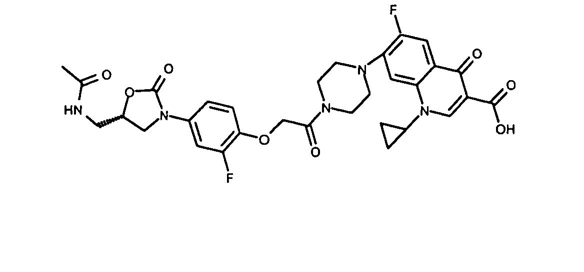

## Claims

**1.** Compounds of formula (I) 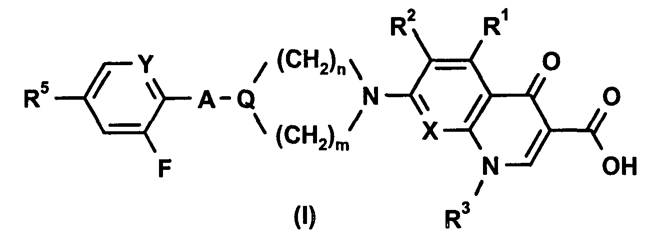 wherein
A is an alkylene group, an alkenylene group, an alkynylene group, a heteroalkylene group, a cycloalkylene group, a heterocycloalkylene group, an arylene group or a heteroarylene group all of which groups may be substituted;
Q is nitrogen or CR⁴;
X is CR⁷ or N;
Y is CR⁶ or N;
n is 1, 2 or 3;
m is 1, 2 or 3;
R¹ is H, F, Cl, Br, I, OH, NH₂, an alkyl group or a heteroalkyl group;
R² is H, F or Cl;
R³ is H, an alkyl group, an alkenyl group, an alkinyl group, a heteroalkyl group, a cycloalkyl group, a heterocycloalkyl group, an aryl group, a heteroaryl group, an alkylaryl group or a heteroarylalkyl group; all of which may be substituted with one, two or more halogen atoms like F or Cl or amino groups;
R⁴ is hydrogen, hydroxy, a group of formula OPO₃H₂ or OSO₃H or a heteroalkyl group carrying at least one OH, NH₂, SO₃H, PO₃H₂ or COOH group;
R⁵ is selected from the following groups: 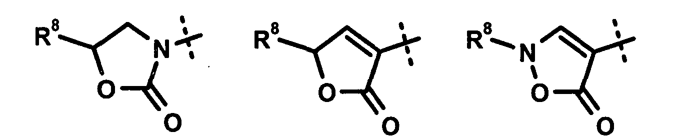 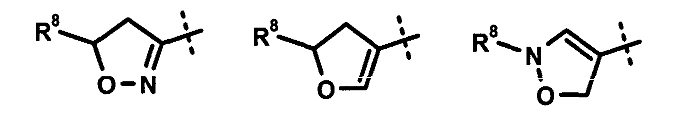 R⁶ is H, F, Cl or OMe;
R⁷ is H, F, Cl, OH, NH₂, an alkyl group or a heteroalkyl group, or
R³ and R⁷ can be linked via an alkylene, an alkenylene or a heteroalkylene group or be a part of a cycloalkylene or heterocycloalkylene group; in case R³ is no H and R⁵ is no H, F, OH, NH₂ or Cl; and
R⁸ is a C₁₋₆ heteroalkyl or a heteroarylalkyl group;
or a pharmacologically acceptable salt, solvate, hydrate or formulation thereof.

**2.** Compounds according to claim 1, wherein R¹ is H.

**3.** Compounds according to claim 1 or 2, wherein R² is F or H.

**4.** Compounds according to any one of claims 1 to 3, wherein R³ is an ethyl, a 2-propyl, a C₃-C₆ cycloalkyl, a phenyl or a pyridyl group; all of which may be substituted with one, two or more fluorine atoms or amino groups.

**5.** Compounds according to any one of claims 1 to 4, wherein R³ is a cyclopropyl group.

**6.** Compounds according to any one of claims 1 to 5, wherein R⁴ is hydroxy or a group of formula OSO₃H, OPO₃H₂, OCH₂OPO₃H₂, OCOCH₂CH₂COOH or an ester of a naturally occurring amino acid or a derivative thereof.

**7.** Compounds according to any one of claims 1 to 6, wherein R⁸ is a group of the formula -CH₂NHCOCH=CHAryl, - CH₂Oheteroaryl (especially -oxa-3-oxazol), -CH₂NHSO₂Me, -CH₂NHCOOMe, -CH₂NHCS₂Me, -CH₂NHCSNH₂, -CH₂NHCSOMe or - NHCOMe.

**8.** Compounds according to any one of claims 1 to 7, wherein R⁵ has the following structure:

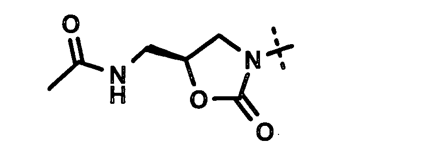

**9.** Compounds according to any one of claims 1 to 8, wherein R³ and R⁷ together form a bridge of the formula -O-CH₂-N(Me)- or O-CH₂-CH(Me)-, wherein, the preferred stereochemistry at the chiral center is the one giving the S configuration in the final compound.

**10.** Compounds according to any one of claims 1 to 8, wherein R⁷ is H, F, Cl or a methoxy group which may be substituted by one, two or three fluorine atoms.

**11.** Compounds according to any one of claims 1 to 10, wherein X is N or CH.

**12.** Compounds according to any one of claims 1 to 11, wherein Y is CH.

**13.** Compounds according to any one of claims 1 to 12, wherein A is C₁₋₆ alkyl, C₂₋₆ alkenyl, C₂₋₆ alkynyl, C₁₋₆ heteroalkyl, cyclopropyl, epoxide, aziridine, thioepoxide, lactame or lactone, all of which groups may be subsitituted.

**14.** Pharmaceutical compositions containing a compound according to any one of Claims 1 to 13 and optionally carriers and/or adjuvants and/or diluents.

**16.** Use of a compound, a pharmaceutical composition or a prodrug according to any one of Claims 1 to 15 for the manufacture of medicaments for the treatment of bacterial infections.
